(19) Europäisches Patentamt

European Patent Office

Office européen des brevets

(11) **EP 0 610 471 B1**

## (12) EUROPÄISCHE PATENTSCHRIFT

(45) Veröffentlichungstag und Bekanntmachung des Hinweises auf die Patenterteilung:
**17.04.1996 Patentblatt 1996/16**

(21) Anmeldenummer: **93917658.2**

(22) Anmeldetag: **27.07.1993**

(51) Int. Cl.$^6$: **A61K 7/06**, C08H 5/02, A61K 7/11

(86) Internationale Anmeldenummer: **PCT/EP93/01990**

(87) Internationale Veröffentlichungsnummer:
**WO 94/05249 (17.03.1994 Gazette 1994/07)**

(54) **MITTEL ZUR FESTIGUNG DER HAARE AUF DER BASIS VON LIGNIN ODER LIGNINDERIVATEN SOWIE DIHYDROXYPROPYLLIGNIN**

HAIR FIXER BASED ON LIGNIN OR LIGNIN DERIVATIVES AND DIHYDROXYPROPYL LIGNIN

AGENT DE FIXATION CAPILLAIRE, A BASE DE LIGNINE OU DE DERIVES DE LIGNINE AINSI QUE DE DIHYDROXYPROPYLLIGNINE

(84) Benannte Vertragsstaaten:
**DE ES FR GB IT**

(30) Priorität: **29.08.1992 DE 4228897**

(43) Veröffentlichungstag der Anmeldung:
**17.08.1994 Patentblatt 1994/33**

(73) Patentinhaber: **Wella Aktiengesellschaft D-64274 Darmstadt (DE)**

(72) Erfinder:
- **LANG, Günther, Dr.**
  **D-64354 Reinheim (DE)**
- **CLAUSEN, Thomas, Dr.**
  **D-64665 Alsbach (DE)**
- **TITZE, Hans-Jürgen**
  **D-64401 Gross-Bieberau (DE)**
- **STEINBRECHT, Karin, Dr.**
  **D-64372 Ober-Ramstadt (DE)**
- **KEIL, Wolfgang, Dr.**
  **D-60386 Frankfurt (DE)**

(56) Entgegenhaltungen:
CH-A- 232 099   CN-A-85 107 583
US-A- 4 803 255

- EUROPEAN POLYMER JOURNAL Bd. 24, Nr. 9, 1988, EXETER,DEVON Seiten 843 - 847 XP000008816 DOURNEL P. ET AL. 'SYNTHESIS AND POLYMERIZATIONOF LIGNIN MACROMONOMERS - I. ANCHORING OF POLYMERIZABLE GROUPS ON LIGNIN MODEL COMPOUNDS'
- JOURNAL OF APPLIED POLYMER SCIENCE Bd. 29, Nr. 5, Mai 1984, NEW YORK, USA Seiten 1815 - 1830 W.G. GLASSER ET AL. 'ENGINEERING PLASTICS FROM LIGNIN. II. CHARACTERIZATION OF HYDROXYALKYL LIGNIN DERIVATIVES' in der Anmeldung erwähnt
- WOOD AND AGRICULTURAL RESIDUES-RESEARCH ON USE FOR FEED, FUELS AND CHEMICALS 1983, TEXAS Seiten 149 - 166 GLASSER W.G. ET AL. 'SYNTHESIS, STRUCTURE, AND SOME PROPERTIES OF HYDROXYPROPYL LIGNINS' in der Anmeldung erwähnt

**Beschreibung**

Die Erfindung betrifft ein Mittel zur Festigung der Haare, welches schwefelfrei gewonnenes Lignin und/oder mindestens ein schwefelfreies Ligninderivat enthält sowie das neue schwefelfreie Ligninderivat Dihydroxypropyllignin.

Mittel zur Festigung der Haare bestehen üblicherweise aus Lösungen von filmbildenden natürlichen oder synthetischen Polymeren. Als Polymere werden beispielsweise anionische Polymere wie Acrylsäure- oder Methacrylsäure-Homopolymere oder -Copolymere, Copolymerisate aus Acrylsäure und Acrylamiden und Copolymere auf der Basis von Alkylvinylethern und Maleinsäuremonoalkylestern, amphotere Polymere wie Copolymerisate aus Octylacrylamid, Acrylat und Butylaminoethylmethacrylat sowie nichtionische Polymere wie Vinylpyrrolidon-Homopolymere, Vinylpyrrolidon-Vinylacetat-Copolymere und Copolymerisate aus Vinylpyrrolidon, Vinylacetat und Vinylpropionat verwendet.

Für die Verwendung in Mitteln zur Festigung der Haare geeignete Polymere müssen eine Vielzahl von Anforderung erfüllen. So müssen sie zum Beispiel in Alkoholen und Alkohol-Wasser-Gemischen gut löslich sowie mit den üblicherweise verwendeten Aerosol-Treibmitteln verträglich sein.

Weiterhin müssen sie auf dem Haar einen klaren, glänzenden Film bilden und dem Haar einen festen und zugleich flexiblen Halt geben ohne den natürlichen Griff des Haares zu beeinträchtigen. Ebenfalls müssen diese Polymere gegen Luftfeuchtigkeit beständig sein, damit auch bei hoher Luftfeuchtigkeit ihre Festigungseigenschaften erhalten bleiben und das Haar sich nicht klebrig anfühlt. Gleichzeitig muß jedoch auch gewährleistet sein, daß diese polymere leicht und rückstandsfrei aus dem Haar wieder ausgewaschen werden können, beziehungsweise durch Ausbürsten entfernbar sind.

Zur Erzielung der vorstehend aufgeführten Eigenschaften müssen diese Polymere häufig mit bestimmten Zusatzstoffen, wie zum Beispiel Weichmachern oder hydrophobierenden Substanzen, kombiniert werden. Ebenfalls ist es üblich, bei freie Säuregruppen enthaltenden Polymeren die Auswaschbarkeit durch eine Neutralisierung der Säuregruppen zu verbessern. Diese Maßnahmen erniedrigen jedoch die Härte der Polymerfilme.

Synthetische haarfestigende Polymere werden bevorzugt durch radikalische Polymerisation von monomeren petrochemischen Erzeugnissen, also aus nicht erneuerbaren Rohstoffen, hergestellt. Zudem können synthetische Polymere Verunreinigungen von Monomeren, Polymerisationsstoppern und Polymerisationsregulatoren enthalten, die toxikologisch bedenklich sein können.

Natürliche Polymere weisen die vorstehend geschilderten Nachteile synthetischer Polymere nicht auf. Als haarfestigende natürliche Polymere kommen insbesondere Schellack, Polysaccharide beziehungsweise Derivate von Polysacchariden, insbesondere Cellulosederivate in Betracht. Während Haarfestiger auf Schellackbasis schlecht auszuwaschen und auszubürsten sind, können Polysacchacid-Lösungen aufgrund ihrer hohen Viskosität nicht in Haarsprays verwendet werden.

Obwohl eine Vielzahl von natürlichen und synthetischen Polymeren mit unterschiedlichen Eigenschaften bekannt sind, ist es bisher nicht gelungen, ein Mittel zur Festigung der Haare zur Verfügung zu stellen, das in allen Eigenschaften - insbesondere aber bezüglich der Festigungswirkung, der Auswaschbarkeit, der Ausbürstbarkeit sowie der Beständigkeit gegenüber Luftfeuchtigkeit - völlig zufriedenstellend ist.

Es bestand daher die Aufgabe, ein Mittel zur Festigung der Haare zur Verfügung zu stellen, das die Nachteile der bisher bekannten Mittel nicht aufweist, das heißt, sowohl eine gute Festigung der Haare bewirkt, als auch beständig gegen Luftfeuchtigkeit ist und gleichzeitig aus dem Haar leicht ausgewaschen werden kann.

Überraschenderweise wurde nunmehr gefunden, daß durch ein Mittel zur Festigung der Haare mit einem Gehalt an schwefelfrei gewonnenem Lignin und/oder mindestens einem schwefelfreien Ligninderivat alle an ein solches Mittel gestellten Anforderungen in hervorragenden Weise erfüllt werden.

Gegenstand der vorliegenden Erfindung ist daher ein Mittel zur Festigung der Haare, welches dadurch gekennzeichnet ist, daß es schwefelfrei gewonnenes Lignin und/oder mindestens ein schwefelfreies Ligninderivat enthält.

Schwefelfrei gewonnenes Lignin wird beispielsweise nach dem Organocell-Verfahren (Firma Organocell, Gesellschaft für Zellstoff- und Umwelttechnik mbH, München) durch Extraktion von Holz mittels eines Methanol-Wasser-Gemisches bei der Zellstoffproduktion erhalten. Die in der Papierindustrie überwiegend gebräuchlichen Verfahren zur Zellstoffproduktion, das Sulfit- und das Sulfatverfahren, liefern schwefelhaltiges Lignin.

Das schwefelfrei gewonnene Rohlignin wird für den Einsatz in dem erfindungsgemäßen Mittel vorzugsweise gereinigt, indem man es mit einem organischen Lösungsmittel, vorzugsweise mit einem organischen Keton, wie zum Beispiel Aceton oder Methylethylketon, oder einem Alkohol, wie zum Beispiel Methanol, Propanol, Butanol oder den entsprechenden Isoalkoholen, besonders bevorzugt jedoch mit Ethanol, 2 bis 12 Stunden lang bei 20 bis 40° Celsius rührt. Die in dem organischen Lösungsmittel unlöslichen Rückstände werden sodann durch Filtration oder Zentrifugation abgetrennt und die erhaltene Extraktionslösung durch Abdestillieren des Lösungsmittels zur Trockene eingeengt.

Als schwefelfreies Ligninderivat ist in dem erfindungsgemäßen Mittel zur Festigung der Haare insbesondere mindestens eine der Verbindungen Hydroxypropyllignin, Hydroxybutyllignin und Dihydroxypropyllignin enthalten.

Schwefelfreies Hydroxypropyl- und Hydroxybutyllignin sind bekannt und können beispielsweise nach den in der Literatur bei Leo C.-F. Wu et al. "Engineering Plastics from Lignin. I., Synthesis of Hydroxypropyl Lignin" J. Appl. Pol. Sci. 29 (1984), Seiten 1111 bis 1123, Wofgang G. Glasser et al. "Engineering Plastics from Lignin. II., Characterisation of Hydroxyalkyl Lignin Derivates" J. Appl. Sci. 29 (1984), Seiten 1815 bis 1830, und bei Wolfgang G. Glasser et al. "Synthesis, Structure and some Properties of Hydroxypropyl Lignins" Wood and Agicultural Residues -Research on use for Feed, Fuels and Chemicals (1983), Seiten 149 bis 166, beschriebenen Herstellungsverfahren hergestellt werden.

Die Verbindung Dihydroxypropyllignin, die in den erfindungsgemäßen Mitteln enthalten sein kann, ist neu und daher ebenfalls Gegenstand der Erfindung.

Die Herstellung von Dihydroxypropyllignin erfolgt, in dem man nach dem vorstehend beschriebenen Verfahren gereinigtes, schwefelfrei gewonnenes Lignin in einem organischen Lösungsmittel, vorzugsweise in 50prozentigem wäßrigem Ethanol, löst, mit 2,3-Epoxy-1-propanol bei einer Temperatur von 20 bis 100° Celsius, vorzugsweise bei 40 bis 80° Celsius 2 bis 24 Stunden, vorzugsweise 24 Stunden lang, umsetzt, sodann den Reaktionsansatz durch Abdestillieren des organischen Lösungsmittels zur Trockene einengt, den verbleibenden Reaktionsansatz in Wasser aufnimmt und anschließend den aus Dihydroxypropyllignin bestehenden Niederschlag aus dem Wasser abfiltriert.

Das erfindungsgemäße Mittel enthält bevorzugt 1 bis 10 Gewichtsprozent des schwefelfrei gewonnenen Lignins und/oder mindestens eines schwefelfreien Ligninderivats. In einer besonders bevorzugten Ausführungsform enthält das erfindungsgemäße Mittel, zusätzlich zu dem schwefelfrei gewonnenen Lignin und/oder mindestens einem schwefelfreien Ligninderivat, 1 bis 15 Gewichtsprozent, bevorzugt 1 bis 12 Gewichtsprozent, mindestens eines weiteren natürlichen filmbildenden Polymers und/oder eines synthetischen filmbildenden Polymers.

Besonders bevorzugt beträgt das Gewichtsverhältnis des schwefelfrei gewonnenen Lignins und/oder mindestens eines schwefelfreien Ligninderivates zu dem weiteren natürlichen filmbildenden Polymers und/oder dem synthetischen filmbildenden Polymer 1:20 bis 4:1.

Als natürliche filmbildende Polymere, die zusätzlich in dem erfindungsgemäßen Mittel enthalten sein können, kommen beispielsweise Chitosan, Chitosanderivate, wie zum Beispiel Hydroxypropyl- und Hydroxybutylchitosan, Schellack, Alginate, Gelatine, Pektine und Cellulosederivate, beispielsweise Hydroxypropylcellulose und Hydroxyethylcellulose, in Betracht.

Von den synthetischen filmbildenden Polymeren finden zum Beispiel Polyvinylpyrrolidon, Polyvinylacetat, Polyacrylverbindungen, wie beispielsweise Acrylsäure- oder Methacrylsäurepolymerisate, basische Polymerisate von Estern aus Acrylsäure oder Methacrylsäure mit Aminoalkoholen oder die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung.

Die zusätzlich in dem erfindungsgemäßen Mittel enthaltenen filmbildenden Polymere können, sofern diese Polymere saure Gruppen enthalten, in einer zu 50 bis 100 Prozent mit organischen Aminen oder Alkanolaminen, bevorzugt mit 2-Amino-2-methyl-1-propanol, 2-Amino-1-butanol oder Triisopropylamin, neutralisierten Form eingesetzt werden.

Das erfindungsgemäße Mittel zur Festigung der Haare liegt in Form einer wäßrigen, alkoholischen oder wäßrigalkoholischen Zubereitung, insbesondere als Schaum, Creme, Emulsion, Lotion oder Gel, vor.

Als Alkohol kommen hierbei insbesondere die für kosmetische Zwecke üblicherweise verwendeten Alkohole mit 1 bis 4 Kohlenstoffatomen, wie zum Beispiel Ethanol und Isopropanol, in Betracht. Diese Alkohole können in dem erfindungsgemäßen Mittel in einer Menge von 10 bis 98 Gewichtsprozent, vorzugsweise in einer Menge von 20 bis 90 Gewichtsprozent, enthalten sein.

Das erfindungsgemäße Mittel zur Festigung der Haare kann in einem Druckbehälter mit einem Treibmittel abgefüllt sein und als Aerosol oder Schaum versprüht werden. Es kann auch in Form eines, als mit Hilfe einer geeigneten mechanisch betriebenen Sprühvorrichtung versprühbaren Non-Aerosol-Haarsprays, Non-AerosolSchaumes oder Non-Aerosol-Haarlackes vorliegen.

Unter mechanischen Sprühvorrichtungen sind solche Vorrichtungen zu verstehen, welche das Versprühen einer Flüssigkeit ohne Verwendung eines verflüssigten Treibmittels ermöglichen. Als geeignete mechanische Sprühvorrichtung kann beispielsweise eine Sprühpumpe oder ein mit einem Sprühventil versehener elastischer Behälter, in den das vorstehend beschriebene kosmetische Mittel unter Druck abgefüllt wird, wobei sich der elastische Behälter ausdehnt und aus dem sich das Mittel infolge der Kontraktion des elastischen Behälters bei Öffnen des Sprühventils kontinuierlich entnehmen läßt, verwendet werden.

Wenn das erfindungsgemäße Mittel in Form eines Aerosol-Haarsprays, Aerosol-Schaumes oder Aerosol-Haarlakkes vorliegt, so enthält es zusätzlich 2 bis 80 Gewichtsprozent eines Treibmittels und wird in einen Druckbehälter abgefüllt. Als Treibmittel sind beispielsweise leicht flüchtige Fluorchlorkohlenwasserstoffe, wie zum Beispiel Difluorchlormethan oder Trichlormonofluormethan, Tetrafluordichlorethan oder niedere Alkane, wie zum Beispiel n-Butan, i-Butan und Propan, oder auch Dimethylether sowie ferner bei den in Betracht kommenden Drücken gasförmig vorliegende Treibmittel, wie beispielsweise $N_2$, $N_2O$ und $CO_2$, sowie Gemische der vorstehend genannten Verbindungen geeignet. Unter den vorstehend genannten Treibmitteln sind Dimethylether sowie Mischungen von niederen Alkanen mit Dimethylether bevorzugt, wobei die Verwendung von Dimethylether als Treibmittel besonders bevorzugt ist.

Weiterhin kann das erfindungsgemäße Mittel für Haarfestigungsmittel übliche kosmetische Zusatzstoffe enthalten, wie beispielsweise anionische, kationische, amphotere oder nicht-ionische Netzmittel und Emulgatoren, wie zum Beispiel $C_{12}$- bis $C_{18}$-Alkylethersulfate, Alkyltrimethylammoniumsalze, Alkylpyridiniumsalze, Carboxyderivate von Imidazol, N-Alkylsulfobetain oder Polyglycerylether von gesättigten oder ungesättigten Fettalkoholen und Alkylphenolen, in einer Menge von etwa 0,01 bis 3 Gewichtsprozent, ferner Konservierungsstoffe, wie zum Beispiel Salicylsäure oder Mandelsäure, in einer Menge von 0,01 bis 0,7 Gewichtsprozent sowie Antischuppenwirkstoffe, wie zum Beispiel Zink-Pyridinthion, kosmetische Farbstoffe, wie zum Beispiel Fluorescein-Natriumsalz, weiterhin haarpflegende Wirkstoffe, wie zum Beispiel Fettsäureester, Fettalkohole, Fettsäureglyceride, Lanolinderivate oder Pantothensäure, in einer Menge von etwa 0,01 bis 3 Gewichtsprozent, hydrophobierende Substanzen wie Siliconöle, beispielsweise Polydimethylsiloxane, Polymethylphenylsiloxane oder Cyclomethicon, Weichmacher wie Phtalsäureester oder Alkylzitrate und kämmbarkeitsverbessernde Substanzen, wie zum Beispiel Cetyltrimethylammoniumchlorid, oder kationische Polymere, zum Beispiel kationische Chitosan- oder Cellulosederivate, in einer Menge von 0,01 bis 0,2 Gewichtsprozent sowie ferner Komplexbildner, Schaumstabilisatoren, Puffersubstanzen, Lichtschutzmittel oder Parfümöle in einer Menge von etwa 0,01 bis 0,8 Gewichtsprozent.

Das erfindungsgemäße Mittel zur Festigung der Haare kann gegebenenfalls durch einen Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche für Haarfestiger bekannte, direkt auf das Haar aufziehende kosmetische Farbstoffe, beispielsweise aromatische Nitrofarbstoffe, wie zum Beispiel 1,4-Diamino-2-nitrobenzol, Pikraminsäure, 1-Hydroxy-2-amino-4-nitrobenzol und 1,4-Bis- (2-hydroxyethyl)amino -2-nitro-5-chlorbenzol, Azofarbstoffe, wie zum Beispiel Acid Brown 4 (C.I. 14 805), Anthrachinonfarbstoffe, wie zum Beispiel Disperse Violet 4 (C.I. 61 105) oder Triphenylmethanfarbstoffe, wie zum Beispiel Basic Violet 1 (C.I. 42 535), wobei die Farbstoffe dieser Klassen je nach Art ihrer Substituenten sauren, nichtionogenen oder basischen Charakter haben können. Die Gesamtkonzentration dieser Farbstoffe beträgt hierbei üblicherweise etwa 0,01 bis 2,0 Gewichtsprozent.

Das erfindungsgemäße Mittel zur Festigung der Haare bewirkt, bei gleichzeitig ausgezeichneter Festigung, eine Verbesserung von Glanz und Griff des Haares und ist sowohl leicht ausbürstbar als auch leicht auswaschbar. Weiterhin fühlt sich das mit diesem Mittel behandelte Haar auch bei hoher Luftfeuchtigkeit nicht klebrig an.

Zudem zeigt das erfindungsgemäße Mittel mit den, in einer bevorzugten Ausführungsform enthaltenen, weiteren natürlichen filmbildenden Polymeren und/oder synthetischen filmbildenden Polymeren, einen synergistischen Effekt, der eine Erhöhung der Filmhärte und der Wasserdampfresistenz der Polymerfilme bewirkt.

Die nachfolgenden Beispiele sollen den Gegenstand der Erfindung näher erläutern.

**Beispiele**

**Herstellungsbeispiel 1: Reinigung des schwefelfrei gewonnenen Rohlignins aus Fichte (pina abies)**

30 kg schwefelfrei gewonnenes Rohlignin aus Fichte (pina abies) werden mit 100 l absolutem Ethanol 4 Stunden bei 25° Celsius gerührt. Anschließend wird der entstandene Niederschlag über eine Filterpresse abfiltriert und das Filtrat durch Abdestillieren des Ethanols zur Trockene eingeengt. Man erhält 15 kg in Aceton und Ethanol klar lösliches, schwefelfrei gewonnenes Lignin.

Zur Bestimmung der Pendelhärte eines Films aus schwefelfrei gewonnenem Lignin wurden 20 ml einer 3,8 prozentigen Lösung des schwefelfrei gewonnenen Lignins in Ethanol auf einer Glasplatte getrocknet und, nach dreistündigem Vortrocknen bei 105° Celsius und Angleichen an ein Klima von 20° Celsius bei 35 Prozent relativer Feuchte, wurde die Pendelhärte nach König (W. König, "Härtemessung mit dem Pendelhärteprüfer", Farbe und Lack 65 (1959),Seiten 435 bis 433; DIN 53157) bestimmt. Es bildete sich ein gläzender glatter, dunkelbrauner Film aus. Die Wasserdampfaufnahme wurde bei 70 Prozent relativer Luftfeuchte gegenüber 35 Prozent relativer Luftfeuchte ermittelt.

Pendelhärte:           154 s
Wasserdampfaufnahme:   1,7 %

**Herstellungsbeispiel 2: Dihydroxypropyllignin**

25 g des nach Herstellungsbeispiel 1 gereinigten, schwefelfrei gewonnenen Lignins werden in 200 ml 50prozentigem wäßrigen Ethanol gelöst, bei 80° Celsius über einen Zeitraum von 1 Stunde mit 22,5 g 2,3-Epoxy-1-propanol versetzt und weitere 23 Stunden lang bei gleicher Temperatur gerührt. Sbdann wird das erhaltene Reaktionsgemisch durch Abdestillieren des Lösungsmittels zur Trockene eingeengt und der verbleibende Rückstand in 500 ml Wasser aufgenommen. Der entstandene wasserunlösliche Niederschlag wird abfiltriert, mehrmals mit Wasser gewaschen und anschließend bei 50° Celsius im Vakuum getrocknet. Man erhält 22 g Dihydroxypropyllignin Form eines mittelbraunen Pulvers, welches in Methanol, 96-prozentigem Ethanol und Tetrahydrofuran löslich, in Wasser jedoch unlöslich ist.

Pendelhärte und Wasserdampfaufnahme wurden nach den in Herstellungsbeispiel 1 geschilderten Bedingungen bestimmt. Es bildete sich ein glänzender, glatter, dunkelbrauner, jedoch unebener Film aus.

Pendelhärte:           152 ± 14 s
Wasserdampfaufnahme:   2,06%

**Herstellungsbeispiel 3: Hydroxypropyllignin**

50 g des nach Herstellungsbeispiel 1 gereinigten, schwefelfrei gewonnenen Lignins werden mit 400 ml Ethylenglykoldimethylether, 36,3 g 42-prozentiger Natronlauge und 260 ml Propylenoxid im Autoklaven bei 1,47 bar 24 Stunden lang bei 70 °Celsius gerührt. Der Reaktionsansatz wird sodann auf Raumtemperatur abgekühlt, mit konzentrierter Salzsäure auf einen pH-Wert = 7 eingestellt und zur Trockene eingeengt.

Der verbliebene Rückstand wird in 1000 ml wasserfreiem Ethanol aufgenommen und filtriert. Nach dem Abdestillieren des Ethanols erhält man 51,1 g Hydroxypropyllignin in Form eines hellbraunen Pulvers, das in Ethanol, Methanol, Isopropanol, Aceton und Tetrahydrofuran löslich, in Wasser jedoch unlöslich ist.

Die Pendelhärte und Wasserdampfaufnahme wurde gemäß den unter Herstellungsbeispiel 1 genannten Bedingungen bestimmt. Es bildete sich ein glänzender, glatter, dunkelbrauner Film aus.

Pendelhärte: 157 s
Wasserdampfaufnahme: 3,8 %

**Herstellungsbeispiel 4: Hydroxybutyllignin**

50 g des nach Herstellungsbeispiel 1 gereinigten, schwefelfrei gewonnenen Lignins werden mit 400 ml Ethylenglykoldimethylether, 36,3 g 42-prozentiger Natronlauge und 260 ml Butylenoxid im Autoklaven bei 1,47 bar 24 Stunden lang bei 70 °Celsius gerührt. Der Reaktionsansatz wird sodann auf Raumtemperatur abgekühlt, mit konzentrierter Salzsäure auf einen pH-Wert = 7 eingestellt und zur Trockene eingeengt.

Der verbliebene Rückstand wird in 1000 ml wasserfreiem Ethanol aufgenommen und filtriert. Nach dem Abdestillieren des Ethanols erhält man 39,5 g Hydroxybutyllignin in Form eines mittelbraunen Pulvers, das in Methanol, Ethanol und Tetrahydrofuran löslich, in Wasser jedoch unlöslich ist.

Die Pendelhärte und Wasserdampfaufnahme wurde gemäß den unter Herstellungsbeispiel 1 genannten Bedingungen bestimmt. Es bildete sich ein glänzender, glatter, dunkelbraunker, leicht unebener Film aus.

Pendelhärte: 144 ± 8 s
Wasserdampfaufnahme: 1,84 %

**Analyse der Ligninderivate der Beispiele 2 bis 4**

Die thermischen Abbauprodukte der Ligninderivate aus den Beispielen 2 bis 4 wurden gaschromatisch getrennt und anschließend massenspektoskopisch identifiziert (vgl. "Geräte-Parameter zur Pyrolyse-GC/MS von Ligninderivaten der Beispiele 2 bis 4"). Die Spaltprodukte wurden durch GC/MS der entsprechenden Referenzsubstanzen bestätigt.

**Geräte-Parameter zur Pyrolyse-GC/MS von Lignin-Derivaten der Beispiele 2 bis 4**

1) Pyrolysator:

Pyrola-9 (Pyrol AB, Lund, Schweden)

Pyrolyse-Temperatur: 600 °C

2) Gaschromatograph:

Kapillar-Gaschromatograph HP 5890A mit split/splitless-Injektor (Hewlett-Packard)

Vordruck: 5,0 bar
Säulenvordruck: 1,04 bar (104 kPa)
Säulendurchfluß: 1,4 ml/min
Septumspülung: 3,0 ml/min
Split-Verhältnis: 1 : 35
Säulentemperatur: 40 °C/5 min/20 °C pro min/250 °C/25 min
Injektortemperatur: 250 °C
Trägergas: Helium

3) Kapillarsäule

| | |
|---|---|
| Typ: | WCOT Fused Silica (Chromapack Nr. 7752) |
| Flüssige Phase: | CP-Sil-19 CB |
| Filmdicke: | 0,20 µm |
| Innerer Durchmesser: | 0,32 mm |
| Äußerer Durchmesser: | 0,45 mm |
| Länge: | 50m |

4) Massenspektrometer:

Ion Trap Detektor ITD 700 (Finnigan MAT)
Ionisierung: Elektronenstoß-Ionisierung bei 70 eV

Die Proben der Ligninderivate der Beispiele 2 bis 4 ergaben bei der Pyrolyse, neben den Abbauprodkten des Lignin-Grundgerüstes, ermittelt durch Pyrolyse von Lignin aus Beispiel 1, charakteristische Spaltprodukte, die bei thermischen Abbau von Lignin aus Beispiel 1 nicht auftraten.

Die ermittelten thermischen Spaltprodukte lassen sich in drei homologe Reihen teilen (vgl. Tabelle 1), die jeweils die Reaktionsprodukte der gleichen thermischen Umsetzungen der Ligninderivate aus den Beispielen 2 bis 4 verkörpern.

**Tabelle 1: Spaltprodukte der Ligninderivate aus den Beispielen 2 bis 4:**

| Derivat | Lignin-O-CH$_2$-CH-CH$_2$OH<br>$\mid$<br>OH<br>(Beispiel 2) | Lignin-O-CH$_2$-CH-CH$_3$<br>$\mid$<br>OH<br>(Beispiel 3) | Lignin-O-CH$_2$-CH-CH$_2$-CH$_3$<br>$\mid$<br>OH<br>(Beispiel 4) |
|---|---|---|---|
| Spaltprodukte Reihe 1 | H$_3$C-CH-CH$_2$OH<br>$\mid$<br>OH | H$_3$C-CH-CH$_3$<br>$\mid$<br>OH | H$_3$C-CH-CH$_2$-CH$_3$<br>$\mid$<br>OH |
| Spaltprodukte Reihe 2 | H$_3$C-C-CH$_2$OH<br>$\parallel$<br>O | H$_3$C-C-CH$_3$<br>$\parallel$<br>O | H$_3$C-C-CH$_2$-CH$_3$<br>$\parallel$<br>O |
| Spaltprodukte Reihe 3 | HOH$_2$C-CH-CH$_2$OH<br>$\mid$<br>OH | HOH$_2$C-CH-CH$_3$<br>$\mid$<br>OH | HOH$_2$C-CH-CH$_2$-CH$_3$<br>$\mid$<br>OH |
|  | H$_2$C——CH-CH$_2$OH<br>$\diagdown$  $\diagup$<br>O | | |

EP 0 610 471 B1

| | Ligninderivat | Spaltprodukt MS (70 eV): m/e (Prozent) |
|---|---|---|
| Reihe 1: | Dihydroxypropyllignin | 1,2-Propandiol 77 (15; $M^+$+1) |
| | Hydroxypropyllignin | 2-Propanol 59 (3; $M^+$-H) |
| | Hydroxybutyllignin | 2-Butanol 73 (4; $M^+$-H) |
| Reihe 2: | Dihydroxypropyllignin | Hydroxyaceton 75 (3; $M^+$+1) |
| | Hydroxypropyllignin | Aceton 58 (9; $M^+$) |
| | Hydroxybutyllignin | 2-Butanon 72 (10; $M^+$) |
| Reihe 3: | Dihydroxypropyllignin | Glycerin 93 (25; $M^+$+1) |
| | Hydroxypropyllignin | 1,2-Propandiol 77 (15; $M^+$+1) |
| | Hydroxybutyllignin | 1,2-Butandiol 91 (11; $M^+$+1) |

Darüberhinaus wurde für Dihydroxypropyllignin als weiteres Spaltprodukt Glycid ermittelt,

**Beispiele für kosmetische Mittel**

| Beispiel 5: | Aerosol-Haarspray |
|---|---|
| 1,0 g | schwefelfrei gewonnenes Lignin nach Herstellungsbeispiel 1 |
| 9,0 g | Vinylpyrrolidon-Vinylacetat-Copolymer |
| 0,1 g | Polydimethylsiloxan |
| 0,2 g | Paraffinöl |
| 89,7 g | Ethanol |
| 100,0 g | |

| Beispiel 6: | Aerosol-Haarspray |
|---|---|
| 10,0 g | Hydroxypropyllignin nach Herstellungsbeispiel 3 |
| 0,1 g | Polydimethylsiloxan |
| 0,3 g | Paraffinöl |
| 89,6 g | Ethanol |
| $\overline{100,0 \text{ g}}$ | |

| Beispiel 7: | Aerosol-Haarspray |
|---|---|
| 5,0 g | Hydroxybutyllignin nach Herstellungsbeispiel 4 |
| 5,0 g | Vinylpyrrolidon-Vinylacetat-Copolymer |
| 0,1 g | Polydimethylsiloxan |
| 0,2 g | Paraffinöl |
| 89,7 g | Ethanol |
| $\overline{100,0 \text{ g}}$ | |

| Beispiel 8: | Aerosol-Haarspray |
|---|---|
| 2,0 g | Schwefelfrei gewonnes Lignin nach Herstellungsbeispiel 1 |
| 2,0 g | Hydroxypropylcellulose |
| 0,2 g | Parfümöl |
| 45,8 g | Ethanol |
| 50,0 g | Dimethylether |
| $\overline{100,0 \text{ g}}$ | |

Die Mittel gemäß den Beispielen 5, 6, 7 und 8 können beispielsweise mit Dimethylether als Treibmittel in einem Mischungsverhältnis von 45:55 in geeignete Sprühbehältnisse abgefüllt werden.

Bei allen drei Rezepturen wird nach der Anwendung ein guter Halt der Frisur bei natürlichem Griff und Aussehen erhalten.

| Beispiel 9: | Non-Aerosol-Haarspray |
|---|---|
| 3,8 g | Schwefelfrei gewonnenes Lignin nach Herstellungsbeispiel 1 |
| 0,1 g | Parfümöl |
| 0,1 g | Polydimethylsiloxan mit einer Viskosität von 20 $mm^2 \cdot s^{-1}$ bei 25 °Celsius |
| 96,0 g | Ethanol |
| $\overline{100,0 \text{ g}}$ | |

| Beispiel 10: | Non-Aerosol-Haarspray |
|---|---|
| 3,8 g | Hydroxypropyllignin nach Herstellungsbeispiel 1 |
| 0,1 g | Parfümöl |
| 0,1 g | Polydimethylsiloxan mit einer Viskosität von 20 mm$^2 \cdot$ s$^{-1}$ bei 25 °Celsius |
| 96,0 g | Ethanol |
| $\overline{100,0\ g}$ | |

| Beispiel 11: | Non-Aerosol-Haarspray |
|---|---|
| 3,8 g | Hydroxybutyllignin nach Herstellungsbeispiel 4 |
| 0,1 g | Parfümöl |
| 0,1 g | Polydimethylsiloxan mit einer Viskosität von 20 mm$^2 \cdot$ s$^{-1}$ bei 25 °Celsius |
| 96,0 g | Ethanol |
| $\overline{100,0\ g}$ | |

| Beispiel 12: | Non-Aerosol-Haarspray |
|---|---|
| 3,8 g | Schwefelfrei gewonnenes Lignin nach Herstellungsbeispiel 1 |
| 0,1 g | Parfümöl |
| 0,1 g | Polydimethylsiloxan mit einer Viskosität von 20 mm$^2 \cdot$ s$^{-1}$ bei 25 °Celsius |
| 80,0 g | Ethanol |
| 16,0 g | Wasser |
| $\overline{100,0\ g}$ | |

| Beispiel 13: | Non-Aerosol-Haarspray |
|---|---|
| 3,8 g | Schwefelfrei gewonnenes Lignin nach Herstellungsbeispiel 1 |
| 0,1 g | Parfümöl |
| 0,1 g | Polydimethylsiloxan mit einer Viskosität von 20 mm$^2 \cdot$ s$^{-1}$ bei 25 °Celsius |
| 90,0 g | Ethanol |
| 5,8 g | Wasser |
| 0,2 g | 2-Amino-1-methyl-propanol |
| $\overline{100,0\ g}$ | |

| Beispiel 14: | Non-Aerosol-Haarspray |
|---|---|
| 3,0 g | Schwefelfrei gewonnes Lignin nach Herstellungsbeispiel 1 |
| 2,0 g | Hydroxybutylchitosan |
| 0,2 g | Parfümöl |
| 0,1 g | Polydimethylsiloxan mit einer Viskosität von 20 mm$^2$ · s$^{-1}$ bei 25° Celsius / 5$^{-1}$ |
| 94,7 g | Ethanol |
| $\overline{100{,}0 \text{ g}}$ | |

Die Mittel gemäß den Beispielen 9 bis 14 werden in Behältnisse abgefüllt aus denen das Mittel zur Festigung der Haare mittels einer Sprühpumpe auf das Haar aufgetragen wird.

Die Anwendung der Non-Aerosol-Haarsprays gemäß den Beispielen 9 bis 14 bewirkt einen guten Halt der Frisur bei natürlichem Griff und Aussehen.

| Beispiel 15: | Einlegemittel |
|---|---|
| 1,00 g | Schwefelfrei gewonnenes Lignin nach Herstellungsbeispiel 1 |
| 0,40 g | Parfümöl |
| 0,05 g | Cetyltrimethylammoniumchlorid |
| 45,00 g | Ethanol |
| 53,55 g | Wasser |
| $\overline{100{,}00 \text{ g}}$ | |

| Beispiel 16: | Einlegemittel |
|---|---|
| 1,00 g | Hydroxypropyllignin nach Herstellungsbeispiel 3 |
| 0,40 g | Parfümöl |
| 0,05 g | Cetyltrimethylammoniumchlorid |
| 45,00 g | Ethanol |
| 53,55 g | Wasser |
| $\overline{100{,}00 \text{ g}}$ | |

| Beispiel 17: | Einlegemittel |
|---|---|
| 1,00 g | Hydroxybutyllignin nach Herstellungsbeispiel 4 |
| 0,40 g | Parfümöl |
| 0,05 g | Cetyltrimethylammoniumchlorid |
| 45,00 g | Ethanol |
| 53,55 g | Wasser |
| $\overline{100{,}00 \text{ g}}$ | |

| Beispiel 18: | Einlegemittel |
|---|---|
| 1,00 g | Dihydroxypropyllignin nach Herstellungsbeispiel 2 |
| 0,40 g | Parfümöl |
| 0,05 g | Cetyltrimethylammoniumchlorid |
| 45,00 g | Ethanol |
| 53,55 g | Wasser |
| 100,00 g | |

Die Anwendung der vorstehenden Einlegemittel erfolgt in der für derartige Mittel üblichen Art und Weise, indem man etwa 20 ml des Mittels in zuvor gewaschenes, handtuchtrockenes Haar verteilt, das Haar sodann zur Frisur legt und trocknet. Das erfindungsgemäße Einlegemittel verleiht dem Haar eine hervorragende Festigung und verlängert so den Halt der Frisur.

| Beispiel 19: | Festigendes Gel |
|---|---|
| 1,000 g | Hydroxypropyllignin nach Herstellungsbeispiel 3 |
| 0,875 g | Hydroxypropylcellulose |
| 0,125 g | kationisches Cellulosederivat (z.B. Polymer JR®-400 der Firma Union Carbide Co.) |
| 0,300 g | Parfümöl |
| 40,000 g | Ethanol |
| 57,700 g | Wasser |
| 100,000 g | |

Das in dem Beispiel 19 enthaltene Hydroxypropyllignin kann durch die gleiche Menge schwefelfrei gewonnenes Lignin oder Hydroxybutyllignin ersetzt werden.

| Beispiel 20: | Tönungsfestiger |
|---|---|
| 1,00 g | Hydroxypropyllignin nach Herstellungsbeispiel 3 |
| 0,15 g | 1,4-Di(2'-hydroxyethylamino)-2-nitro-5-chlorbenzol |
| 40,00 g | Ethanol |
| 58,85 g | Wasser |
| 100,00 g | |

Die Anwendung des vorstehenden Tönungsfestigers erfolgt in der für derartige Mittel üblichen Art und Weise, indem man etwa 20 ml des Mittel in zuvor gewaschenes, handtuchtrockenes Haar. Der erfindungsgemäße Tönungsfestiger verleiht dem Haar eine rot-violette Färbung und eine hervorragende Festigung.

**Vergleichsversuche 21 bis 31:**

Die erfindungsgemäßen, eine Kombination aus schwefelfrei gewonnenem Lignin und einem synthetischen filmbildenden Polymer enthaltenden Vergleichsbeispiele 23, 24, 26, 27, 29, 30 und 31 der folgenden Tabelle 1 wurden bezüglich der Pendelhärte und der Wasserdampfaufnahme des erhaltenen Polymerfilms mit dem nur schwefelfrei gewonnenes Lignin enthaltenden erfindungsgemäßen Vergleichsbeispiel 21 und den entsprechenden nicht-erfindungsgemäßen Vergleichsbeispielen 22, 25 oder 28, die jeweils nur ein synthetisches filmbildendes Polymer enthalten, verglichen.

12

Die Pendelhärte und Wasserdampfaufnahme wurde gemäß den unter Herstellungsbeispiel 1 genannten Bedingungen bestimmt. Dazu wurden jeweils 20 ml eines Mittel gemäß den Vergleichsbeispielen 21 bis 31 verwendet.

**Tabelle 1:**

| | | 21 | 22 | 23 | 24 |
|---|---|---|---|---|---|
| Schwefelfrei gewonnenes Lignin nach Herstellungsbeispiel 1 | | 3,8 g | - | 0,6 g | 1,9 g |
| Vinylpyrrolidon-Vinyl-acetat-Vinylpropionat-Copolymer | | - | 3,8 g | 3,2 g | 1,9 g |
| Vinylacetat-Crotonsäure-Vinylneodecanoat-Terpolymer | | - | - | - | - |
| Maleinsäurebutylester-Methylvinylether-Copolymer | | - | - | - | - |
| Ethanol | | 96,2 g | 96,2 g | 96,2 g | 96,2 g |
| | | 100,0 g | 100,0 g | 100,0 g | 100,0 g |
| Pendelhärte | s | 154 | 161 | 179 | 204 |
| Wasserdampfaufnahme | % | 1,7 | 5,9 | 3,5 | 2,5 |

**Tabelle 1:**

|                                                                          |     | 25        | 26        | 27        |
| ------------------------------------------------------------------------ | --- | --------- | --------- | --------- |
| Schwefelfrei gewonnenes Lignin nach Herstellungs- beispiel 1             |     | -         | 0,8 g     | 1,9 g     |
| Vinylpyrrolidon-Vinyl- acetat-Vinylpropionat- Copolymer                  |     | -         | -         | -         |
| Vinylacetat-Crotonsäure- Vinylneodecanoat- Terpolymer                    |     | 3,8 g     | 3,0 g     | 1,9 g     |
| Maleinsäurebutylester- Methylvinylether-Copolymer                        |     | -         | -         | -         |
| Ethanol                                                                  |     | 96,2 g    | 96,2 g    | 96,2 g    |
|                                                                          |     | 100,0 g   | 100,0 g   | 100,0 g   |
| Pendelhärte                                                              | s   | 183       | 193       | 196       |
| Wasserdampfaufnahme                                                      | %   | 1,57      | 1,41      | 1,51      |

## Tabelle 1:

|  |  | 28 | 29 | 30 | 31 |
|---|---|---|---|---|---|
| Schwefelfrei gewonnenes Lignin nach Herstellungs-beispiel 1 |  | – | 0,4 g | 1,2 g | 1,9 g |
| Vinylpyrrolidon-Vinyl-acetat-Vinylpropionat-Copolymer |  | – | – | – | – |
| Vinylacetat-Crotonsäure-Vinylneodecanoat-Terpolymer |  | – | – | – | – |
| Maleinsäurebutylester-Methylvinylether-Copolymer |  | 3,8 g | 3,4 g | 2,6 g | 1,9 g |
| Ethanol |  | 96,2 g | 96,2 g | 96,2 g | 96,2 g |
|  |  | 100,0 g | 100,0 g | 100,0 g | 100,0 g |
| Pendelhärte | s | 177 | 179 | 184 | 186 |
| Wasserdampfaufnahme | % | 2,6 | 2,6 | 2,6 | 2,2 |

Die Vergleichsbeispiele 23 und 24 beziehungsweise 26 und 27 oder 30 und 31 zeigen die synergistische Wirkung der Mittel, welche eine Kombination von schwefelfrei gewonnenen Lignin mit für derartige Mittel üblichen synthetischen filmbildenden Polymeren aufweisen, gegenüber dem nur schwefelfrei gewonnenes Lignin enthaltenden Vergleichsbeispiel 21 und den nicht-erfindungsgemäßen Vergleichsbeispielen 22, 25 und 28, welche nur ein für derartige Mittel übliches filmbildendes synthetisches Polymer enthalten, die sich in einer Erhöhung der Polymerfilmhärte und teilweise überproportionalen Erniedrigung der Wasserdampfaufnahme bemerkbar macht.

## Vergleichsbeispiele 32 bis 43

Die erfindungsgemäßen, eine Kombination aus Hydroxypropyllignin und einem synthetischen filmbildenden Polymer enthaltenden Vergleichsbeispiele 34, 35, 37, 38, 39, 41, 42, und 43 der folgenden Tabelle 2 wurden bezüglich der Pendelhärte und der Wasserdampfaufnahme des erhaltenen Polymerfilms mit dem nur Hydroxypropyllignin enthaltenden erfindungsgemäßen Vergleichsbeispiel 32 und den entsprechenden nicht-erfindungsgemäßen Vergleichsbeispielen 33, 36 und 40, die jeweils nur ein synthetisches filmbildendes Polymer enthalten, verglichen.

Die Pendelhärte und Wasserdampfaufnahme der Polymerfilme wurde wie in den Vergleichsbeispielen 21 bis 31 geschildert ermittelt.

**Tabelle 2:**

|  |  | 32 | 33 | 34 | 35 |
|---|---|---|---|---|---|
| Hydroxypropyllignin nach Herstellungsbeispiel 3 |  | 3,8 g | – | 1,2 g | 1,9 g |
| Vinylpyrrolidon-Vinyl-acetat-Copolymer |  | – | 3,8 g | 2,6 g | 1,9 g |
| Vinylpyrrolidon-Vinyl-acetat-Vinylpropionat-Copolymer |  | – | – | – | – |
| Vinylacetat-Crotonsäure-Copolymer |  | – | – | – | – |
| Ethanol |  | 96,2 g | 96,2 g | 96,2 g | 96,2 g |
|  |  | 100,0 g | 100,0 g | 100,0 g | 100,0 g |
| Pendelhärte | s | 157 | 151 | 166 | 175 |
| Wasserdampfaufnahme | % | 3,8 | 4,6 | 3,85 | 3,5 |

**Tabelle 2:**

|  |  | 36 | 37 | 38 | 39 |
|---|---|---|---|---|---|
| Hydroxypropyllignin nach Herstellungsbeispiel 3 |  | – | 0,4 g | 1,2 g | 1,9 g |
| Vinylpyrrolidon-Vinyl-acetat-Copolymer |  | – | – | – | – |
| Vinylpyrrolidon-Vinyl-acetat-Vinylpropionat-Copolymer |  | 3,8 g | 3,4 g | 2,6 g | 1,9 g |
| Vinylacetat-Crotonsäure-Copolymer |  | – | – | – | – |
| Ethanol |  | 96,2 g | 96,2 g | 96,2 g | 96,2 g |
|  |  | 100,0 g | 100,0 g | 100,0 g | 100,0 g |
| Pendelhärte | s | 139 | 160 | 174 | 181 |
| Wasserdampfaufnahme | % | 5,9 | 5,3 | 4,5 | 3,9 |

## Tabelle 2:

| | | 40 | 41 | 42 | 43 |
|---|---|---|---|---|---|
| Hydroxypropyllignin nach Herstellungsbeispiel 3 | | – | 0,4 g | 1,2 g | 1,9 g |
| Vinylpyrrolidon-Vinyl-acetat-Copolymer | | – | – | – | – |
| Vinylpyrrolidon-Vinyl-acetat-Vinylpropionat-Copolymer | | – | – | – | – |
| Vinylacetat-Crotonsäure-Copolymer | | 3,8 g | 3,4 g | 2,6 g | 1,9 g |
| Ethanol | | 96,2 g | 96,2 g | 96,2 g | 96,2 g |
| | | 100,0 g | 100,0 g | 100,0 g | 100,0 g |
| Pendelhärte | s | 116 | 127 | 144 | 171 |
| Wasserdampfaufnahme | % | 2,6 | 2,8 | 2,8 | 3,1 |

Die Vergleichsbeispiele 34 und 35 beziehungsweise 37, 38 und 39 oder 41, 42 und 43 zeigen die synergistische Wirkung der Mittel, welche eine Kombination von Hydroxypropyllignin mit für derartige Mittel üblichen synthetischen filmbildenden Polymeren aufweisen, gegenüber dem nur Hydroxypropyllignin enthaltenden Vergleichsbeispiel 32 und den nicht-erfindungsgemäßen Vergleichsbeispielen 33, 36 und 40, welche nur ein für derartige Mittel übliches filmbildendes synthetisches Polymer enthalten, die sich in einer Erhöhung der Polymerfilmhärte und teilweise überproportionalen Erniedrigung der Wasserdampfaufnahme bemerkbar macht.

Sämtliche in der Anmeldung angegebenen Prozentzahlen stellen Gewichtsprozente dar.

### Patentansprüche

1. Mittel zur Festigung der Haare, dadurch gekennzeichnet, daß es schwefelfrei gewonnenes Lignin und/oder mindestens ein schwefelfreies Ligninderivat enthält.

2. Mittel nach Anspruch 1, dadurch gekennzeichnet, daß das schwefelfreie Ligninderivat ausgewählt ist aus Hydroxypropyllignin, Hydroxybutyllignin und Dihydroxypropyllignin.

3. Mittel nach Anspruch 1 oder 2, dadurch gekennzeichnet, daß es zusätzlich 1 bis 15 Gewichtsprozent mindestens eines weiteren natürlichen filmbildenden Polymers und/oder eines synthetischen filmbildenden Polymers enthält.

4. Mittel nach Anspruch 3, dadurch gekennzeichnet, daß das Gewichtsverhältnis des schwefelfrei gewonnenen Lignins und/oder mindestens eines schwefelfreien Ligninderivates zu dem weiteren natürlichen filmbildende Polymer und/oder synthetischen filmbildenden Polymer 1:20 bis 4:1 beträgt.

5. Mittel nach einem der Ansprüche 1 bis 4, dadurch gekennzeichnet, daß es in Form einer wäßrigen, alkoholischen oder wäßrig-alkoholischen Zubereitung vorliegt.

**6.** Mittel nach einem der Ansprüche 1 bis 5, dadurch gekennzeichnet, daß es 2 bis 80 Gewichtsprozent eines Treibmittels enthält und, in einen Druckbehälter abgefüllt, als Aerosol oder Schaum versprüht wird.

**7.** Mittel nach einem der Ansprüche 1 bis 5, dadurch gekennzeichnet, daß es in Form eines Non-Aerosol-Haarsprays, Non-Aerosol-Schaumes oder Non-Aerosol-Haarlackes vorliegt.

**8.** Mittel nach einem der Ansprüche 1 bis 7, dadurch gekennzeichnet, daß es zusätzlich mindestens einen kosmetischen Farbstoff in einer Konzentration von 0,01 bis 2,0 Gewichtsprozent enthält und in Form eines Farb- oder Tönungsfestigers vorliegt.

**9.** Dihydroxypropyllignin.

## Claims

**1.** Hair fixing agent, characterised in that it contains lignin obtained without sulphur and/or at least one sulphur-free lignin derivative.

**2.** Agent according to Claim 1, characterised in that the sulphur-free lignin derivative is selected from among hydroxypropyl lignin, hydroxybutyl lignin and dihydroxyypropyl lignin.

**3.** Agent according to Claim 1 or Claim 2, characterised in that it additionally contains 1 to 15 weight per cent of at least one further natural film-forming polymer and/or a synthetic film-forming polymer.

**4.** Agent according to Claim 3, characterised in that the weight ratio of the lignin obtained without sulphur and/or at least one sulphur-free lignin derivative to the further natural film-forming polymer and/or synthetic film-forming polymer is 1:20 to 4:1.

**5.** Agent according to any one of Claims 1 to 4, characterised in that it is in the form of an aqueous, alcoholic or aqueous-alcoholic preparation.

**6.** Agent according to any one of Claims 1 to 5, characterised in that it contains 2 to 80 weight per cent of a propellant, and, decanted into a pressurised container, is sprayed as an aerosol or foam.

**7.** Agent according to any one of Claims 1 to 5, characterised in that it is in the form of a non-aerosol hairspray, a non-aerosol foam or a non-aerosol hair lacquer.

**8.** Agent according to any one of Claim 1 to 7, characterised in that it additionally contains at least one cosmetic dye in a concentration of from 0.01 to 2.0 weight per cent and is in the form of a colour or tint fixative.

**9.** Dihydroxypropyl lignin.

## Revendications

**1.** Produit de fixation des cheveux, caractérisé en ce qu'il contient une lignine amenée à un état non soufré et/ou au moins un dérivé non soufré de la lignine.

**2.** Produit selon la revendication 1, caractérisé en ce que le dérivé non soufré de la lignine est compris dans le groupe composé de l'hydroxypropyllignine, de l'hydroxybutyllignine, et la dihydroxyproyllignine.

**3.** Produit selon la revendication 1 ou 2, caractérisé en ce qu'il comprend en plus de 1 à 15 % en poids d'au moins un autre polymère filmogène naturel et/ou un polymère synthétique filmogène.

**4.** Produit selon la revendication 3, caractérisé en ce que le rapport en poids de la lignie amenée à l'état non soufrée et/ou au moins d'un dérivé non souffré de la lignine et l'autre polymère filmogène naturel et/ou le polymère synthétique filmogène est compris entre 1:20 et 4:1.

**5.** Produit sel on l'une des revendications 1 à 4, caractérisé en ce qu'il se présente sous la forme d'une préparation aqueuse, alcoollique ou alcoolo-aqueuse.

6. Produit selon l'une des revendications 1 à 5, caractérisé en ce qu'il contient de 2 à 80 % en poids d'un produit moteur, est conditionné dans récipient sous pression, à titre d'aérosol ou de mousse.

7. Produit selon l'une des revendications 1 à 5, caractérisé en ce qu'il se présente sous la forme d'un spray pour cheveux de type non aérosol, d'une mousse non aérosol ou d'une laque pour cheveux, non aérosol.

8. Produit selon l'une des revendications 1 à 7, caractérisé en ce qu'il contient en plus au moins un colorant cosmétique, en une concentration allant de 0,01 à 2,0 % en poids et se présente sous la forme d'un fixateur colorant ou de nuance.

9. Dihydroxypropyllignine.